# NEUE EUROPÄISCHE PATENTSCHRIFT

(11) **EP 0 421 454 B2**
(45) Veröffentlichungstag und Bekanntmachung des Hinweises auf die Entscheidung über den Einspruch: **05.04.2000**
(45) Hinweis auf die Patenterteilung: 09.08.1995
(21) Anmeldenummer: 90119112.2
(22) Anmeldetag: 05.10.1990
(51) Int. Cl.: A61L 15/16, A61K 9/70

(54) **Oestrogenhaltiges Wirkstoffpflaster**
Dressing containing estrogen
Pansement médical contenant des oestrogènes

(30) Priorität: 06.10.1989 DE 3933460
(43) Veröffentlichungstag der Anmeldung: 10.04.1991
(73) Patentinhaber: LTS Lohmann Therapie-Systeme GmbH & Co. KG, 56513 Neuwied (DE); SCHWARZ PHARMA AG, D-40789 Monheim/Rhld. (DE)
(72) Erfinder: Hoffmann, Hans-Rainer, Dr., W-5450 Neuwied 22 (DE); Klein, Robert Peter, W-5450 Neuwied 11 (DE); Meconi, Reinhold, Dipl.-Ing. (FH), W-5450 Neuwied 1 (DE); Cordes, Günter, Dr. sc. nat., W-5653 Leichlingen (DE); Wolff, Hans Michael, Dr. Dipl.-Chem., W-4019 Monheim (DE)
(74) Vertreter: Flaccus, Rolf-Dieter, Dr.

(56) Entgegenhaltungen:
- EP-A- 0 072 251
- EP-A- 0 186 019
- EP-A- 0 285 563
- EP-A- 0 416 842
- WO-A-82/00005
- GB-A- 2 094 809
- US-A- 4 789 547

## Beschreibung

Wirkstoffpflaster zur kontrollierten Abgabe von Wirkstoffen an die Haut, bestehend aus einer wirkstoffundurchlässigen Rückschicht, einem den oder die Wirkstoff(e) enthaltenden Haftkleber und einer wiederablösbaren Schutzschicht, sowie ein Verfahren zur Herstellung des Wirkstoffplasters und seine Verwendung zur transdermalen Applikation von Oestrogenen, ihren pharmazeutisch unbedenklichen Derivaten alleine oder in Kombination mit Gestagenen in der Humanmedizin.

Wirkstoffplaster sind auf die Haut aufzubringende selbstklebende galenische Zubereitungen mit festgelegter Applikationsfläche, die einen oder mehrere darin enthaltene Arzneistoffe nach Zeit und Menge kontrolliert an den menschlichen oder tierischen Körper abgeben. Derartige Systeme, die beispielsweise von Y.W. Chieng, Drug Dev. Ind. Pharm. 13, 589-651 (1987) beschrieben sind, haben sich seit Jahren in der Therapie bewährt.

Viele dieser Wirkstoffplaster enthalten die Wirkstoffe fein verteilt in hydrophoben Klebefilmen und stellen demzufolge konzeptionell einfache, serienmäßig herstellbare pharmazeutische Zubereitungen dar.

Übliche Bauformen von transdermalen Systemen, die bereits Eingang in die Praxis gefunden haben, sind:
a) Aufbau aus undurchlässiger Rückschicht und einer gleichzeitig als Arzneistoffreservoir, Haftkleber und Steuereinheit dienenden Schicht,
b) Aufbau aus Rückschicht, Arzneistoffreservoir, Kontrolleinheit und Klebeschicht in räumlicher Trennung,
c) Aufbau aus Rückschicht und mehrschichtig angeordneter arzneistoffhaltiger Matrix, wobei die Wirkstoffkonzentration von Schicht zu Schicht zur Haut hin geringer wird.
d) Aufbau aus Rückschicht und Matrix, wobei die Freisetzung durch die in der Matrix dispergierte arzneistoffhaltige Mikrokapsel kontrolliert wird.

Der therapeutische Fortschritt dieser Systeme gegenüber traditionellen Applikationsformen besteht darin, daß die Wirkstoffe dem Körper nicht stoßweise zugefügt werden wie beispielsweise bei Einnahme von Tabletten, sondern kontinuierlich.

Dadurch wird einerseits die Wirkungsdauer des Arzneistoffes verlängert, zum andern werden Nebenwirkungen durch Vermeidung unnötiger Blutspiegelspitzen weitgehend verhindert.

Arbeitet man eine größere Wirkstoffmenge in ein solches Pflaster ein, als dem Sorptionsvermögen der filmbildenden Pflasterbestandteile entspricht, so muß der Wirkstoff möglichst fein bis amorph in der Klebermatrix verteilt sein, um durch rasches Nachlösen über die Applikationsdauer den Sättigungszustand des Haftklebers weitestgehend aufrechtzuerhalten und auf diese Weise den Grad der Abnahme der Geschwindigkeit der Freisetzung des Wirkstoffes aus dem Pflaster so klein wie möglich zu halten. Üblicherweise wird die Herstellung von Pflasterfilmen so vorgenommen, daß Kleberbestandteile und Wirkstoff gemeinsam in einem organischen Lösemittel gelöst und nach Ausstreichen auf großflächige Bahnen getrocknet werden.

Aus der DE-OS 3205258 und der EP 0285563 ist bekannt, Estradiol und Ethanol gleichzeitig in einer Pflasterformulierung zu verabreichen. Dieses Pflaster ist jedoch sehr kompliziert aufgebaut und außerdem sehr aufwendig herzustellen, da die Einzelkomponenten separat hergestellt und dann in einem weiteren Arbeitsgang zu einem Pflaster zusammengefügt werden müssen.

In der WO 87/07138 ist ein Estradiol-Pflaster auf der Basis einer Rückschicht beschrieben, einer den Wirkstoff enthaltenden Matrix und einem Haftkleber, der mit einer entfernbaren Schutzschicht abgedeckt ist. Die Herstellung von Matrix und Haftkleber erfolgen in technologisch sehr aufwendigen Arbeitsgängen durch Homogenisieren, Entgasen, Beschichten, Trocknen und Vereinzeln. In einer Ausführungsform muß die Rückschicht sogar mit einem Haftkleber beschichtet werden, was einen weiteren Arbeitsgang bedingt. Das Zusammenfügen der einzelnen Teile erfolgt in einem separaten Arbeitsgang. Die Herstellung des Pflasters ist also insgesamt sehr aufwendig und kompliziert.

Aus der US-PS 4,624,665 sind Systeme bekannt, die im Reservoir den Wirkstoff in mikroverkapselter Form enthalten. Das Reservoir ist eingebettet zwischen Rückschicht und einer Membran. Der äußere Rand des Systems ist mit einem Haftkleber ausgerüstet. Der Aufbau und die Herstellung dieser Systeme ist sehr kompliziert, da der Wirkstoff mikroverkapselt und in einer flüssigen Phase homogen verteilt werden muß, die dann in weiteren Arbeitsgängen zwischen Rückschicht und Membran eingebettet wird. Zusätzlich muß das System dann mit dem klebenden Rand versehen und mit einer Schutzschicht abgedeckt werden.

Es sind weiterhin aus der EP 186019 Wirkstoffpflaster bekannt, bei denen einer Kautschuk/Klebeharzmasse in Wasser quellbare Poloymere zugesetzt sind und aus denen Estradiol freigesetzt werden kann. Es hat sich jedoch gezeigt, dass die Freisetzung von Estradiol aus diesen Wirkstoffpflastern viel zu gering ist und nicht den therapeutischen Erfordernissen entspricht.

Es ist deshalb Aufgabe der vorliegenden Erfindung, ein Wirkstoffpflaster bereitzustellen, dessen Wirkstofffreisetzung die therapeutischen Erfordernisse erfüllt.

Überraschenderweise wurde die Aufgabe gelöst durch die Bereitstellung eines Wirkstoffpflasters zur kontrollierten Abgabe von Wirkstoffen an die Haut, welches besteht aus einer Rückschicht, einem damit verbundenen wasserunlöslichen Klebefilm aus einem Haftkleber, der in Wasser quellbare Polymere enthält und in dem die Wirkstoffe zumindest teilweise löslich sind, und aus einer den Klebefilm abdeckenden wiederablösbaren Schutzschicht, dadurch gekennzeichnet, daß der Haftkleber auf Homo- und/oder Copolymeren mit mindestens einem Derivat der Acryl- oder Methacrylsäure basiert und als im Kleber teilweise oder vollständig gelöste Wirkstoffe in einer Konzentration von 0,5 bis 10,0 Gew.-% Oestrogene und ihre pharmazeutisch unbedenklichen Derivate alleine oder in Kombination mit Gestagenen enthält.

Es hat sich überraschend gezeigt, daß die Kombination von wasserquellbaren Polymeren mit Polymeren auf Acrylatbasis für die genannten Wirkstoffe Freisetzungsverhältnisse schafft, die über längere Zeit die Abgabe der Wirkstoffe aus den Pflastern in einer Menge gewährleisten, die ein Mehrfaches von der beträgt, die nach dem Stand der Technik freigesetzt wird.

Eine zweckmäßige Ausführungsform kann Stoffe enthalten, die die Kristallisation des Wirkstoffs verzögern oder verhindern und die in einer Konzentration von 0,1 - 20 Gew.-%, vorzugsweise 0,5 - 10 Gew.-% enthalten sind, sowie in Wasser quellbare Polymere in einem Anteil von 0,01 - 10 Gew.-%, vorzugsweise 0,1 - 5 Gew.-%. Die klebrigmachenden Harze liegen vorteilhaft in einer Menge von 0,5 - 50 Gew.-% vor, vorzugsweise 1 bis 20 Gew.-%. Die Dicke des wirkstoffhaltigen Klebefilms kann 0,01 - 0,30 mm, vorzugsweise 0,04 - 0,20 mm betragen,
Als Haftkleber können dabei Homo- und/oder Copolymere mit mindestens einem Derivat der Acryl- oder Methacrylsäure in Form von Lösungen in organischen Lösemitteln Verwendung finden, und zwar in unvernetzbarer oder vernetzbarer Form. Das vernetzende Agenz bewirkt, daß über reaktive Gruppen die Polymerketten miteinander verknüpft werden und so die Kohäsion des Haftklebers erhöht wird.

Die vernetzbaren Haftkleber in Form von organischen Lösungen werden vorzugsweise polymerisiert aus der Kombination folgender Monomerer:
2-Ethylhexylacrylat/n-Butylacrylat/Butylacrylat/Acrylsäure,
2-Ethylhexylacrylat/n-Butylacrylat/Vinylacetat/Acrylsäure,
2-Ethylhexylacrylat/Vinylacetat/Acrylsäure,
2-Ethylhexylacrylat/Vinylacetat/Allylacrylat,
2-Ethylhexylacrylat/Vinylacetat/Divinylbenzol/Acrylsäure,
2-Ethylhexylacrylat/Vinylacetat/Allylmethacrylat/Acrylsäure,
2-Ethylhexylacrylat/Vinylacetat/2-Hydroxyethylacrylat,
2-Ethylhexylacrylat/Vinylacetat/2-Hydroxyethylmethacrylat,
2-Ethylhexylacrylat/Fumarsäure-diethylester/Acrylsäure,
2-Ethylhexylacrylat/Maleinsäure-diethylester/2-Hydroxyethylacrylat.

Als vernetzende Agentien kommen bevorzugt folgende Verbindungen in Frage:
Diphenylmethan-4-diisocyanat, Hexamethylendiisocyanat, Isophorondiisocyanat, Titan-acetylacetonat, Aluminium-acetylacetonat, Eisen-acetylacetonat, Zink-acetylacetonat, Magnesium-acetylacetonat, Zirkon-acetylacetonat, 2-Ethyl-1,3-hexandiol-titanat, Tetraisooctyltitanat, Tetranonyltitanat, polyfunktionelle Propyleniminderivate, veretherte Melaminformaldehydharze, hochmethylierte Urethanharze, Imino-Melaminharze.

Die nicht vernetzbaren Haftkleber in Form von organischen Lösungen können vorteilhaft beispielsweise aus der Kombination folgender Monomerer polymerisiert werden:
2-Ethylhexylacrylat/n-Butylacrylat/Vinylacetat,
2-Ethylhexylacrylat/Vinylacetat,
2-Ethylhexylacrylat/n-Butylacrylat/Vinylacetat/Allylacrylat,
2-Ethylhexylacrylat/n-Butylacrylat/Allylmethacrylat,
2-Ethylhexylacrylat/n-Butylacrylat/Vinylacetat/Divinylbenzol,
2-Ethylhexylacrylat/Fumarsäurediethylester/Allylacrylat,
2-Ethylhexylacrylat/Maleinsäurediethylester/Allylacrylat,
2-Ethylhexylacrylat/n-Butylacrylat/Acrylamid/Vinylacetat/Allylacrylat,
2-Ethylhexylacrylat/n-Butylacrylat/Isobutylacrylat/Vinylacetat/Allylacrylat.

Weiterhin können Haftkleber in Form von wäßrigen Dispersionen verwendet werden. Sie haben ein den Lösemittelhaftklebern vergleichbares Leistungspektrum, besitzen jedoch den Vorteil, daß bei Beschichtung und Trocknung keine brennbaren und giftigen Lösemittel anfallen.
Haftkleber in Form von wässrigen Dispersionen, sog. Dispersionshaftkleber, können z.B. vorteilhaft aus der Kombination folgender Monomer polymerisiert werden:
n-Butylacrylat/Isobutylacrylat/Acrylsäure,
2-Ethylhexylacrylat/n-Butylacrylat/Acrylsäure,
2-Ethylhexylacrylat/n-Butylacrylat/2-Hydroxyethylacrylamid,
2-Ethylhexylacrylat/n-Butylacrylat/Vinylacetat/Acrylamid,
2-Ethylhexylacrylat/n-Butylacrylat/Vinylacetat/2-Hydroxy ethylacrylat,
2-Ethylhexylacrylat/n-Butylacrylat/Allylacrylat/Acrylsäure,
2-Ethylhexylacrylat/n-Butylacrylat/Vinylacetat/Divinylbenzol.

Außerdem können sogenannte Schmelzhaftkleber Verwendung finden, die aus einer Schmelze heraus aufgetragen werden.

Die im erfindungsgemäßen Wirkstoffpflaster in der Klebmasse zugesetzten, in Wasser quellfähigen Polymeren sind Galaktomannane, mikrokristalline Cellulose, Polyglycoside, feinpulverisierte Polyamide, wasserlösliches Polyacrylamid, Carboxyvinylpolymerisate, agar-ähnliche Algenprodukte, Mischpolymerisate aus Methylvinylether und Maleinsäureanhydrid, Dextrin, mikrobiologisch gewonnenes Polysaccharid-Gummi, Ficoll, Methylglucosederivate, Hydroxypropylguar-Gummi, Hydroxymethylpropylcellulose, Polygalacturonsäurederivate wie Pectin und Pectinamid,

Dabei sind besonders bevorzugt Galaktomannane, und/oder solche, die eine Wasserlöslichkeit von ≤ 0,1 Gew.% besitzen mikrokristalline Cellulose.

Als Bestandteile des Haftklebers können außerdem klebrigmachende Harze wie Kolophonium und dessen Derivate Polyterpenharze aus α oder β-Pinen, aliphatische, aromatische oder alkylaromatische Kohlenwasserstoffharze, Melamin-Formaldehyd-Harze, Phenolharze, Hydroabietylalkohol und deren Gemische Verwendung finden.

Weitere Bestandteile des Haftklebers können Kristallisationsverzögerer wie Phthalsäureester, Adipinsäureester, Mono-, Di- und Triglyceride, Ester höherer Fettsauren, langkettige Alkohole und deren Derivate, Derivate des Nonylphenol bzw. des Octylphenol, Derivate von Fettsäuren, Derivate des Sorbit und des Mannit, nichtionogene Tenside, Polyoxyethylenalkylether, Derivate des Rizinusöls, Sitosterin und Polyvinylpyrrolidon sowie weitere dem Fachmann bekannte Stoffe sein.

Die Dicke des wirkstoffhaltigen Klebefilms kann 0,01 - 0,30 mm, vorzugsweise 0,04 - 0,20 mm betragen.

Geeignete Materialien für die wirkstoffundurchlässige Rückschicht sind beispielsweise Polyester, Polyamid, Polyethylen, Polypropylen, Polyurethane, Polyvinylchlorid, und zwar sowohl als sogenannte Solofolien als auch als Sandwichfolien in Kombination von Folien aus verschiedenen dieser Kunststoffe. Diese Folien können eine Dicke von 0,06 - 0,20 mm aufweisen und außerdem mit Aluminium bedampft bzw. mit einer Aluminiumfolie kaschiert sein.

Geeignete Materialien für die wiederablösbare Schutzschicht sind beispielsweise Polyester, Polyethylen und Polypropylen sowie Papiere, die mit diesen Materialien beschichtet und ggf. mit Aluminium bedampft bzw. mit einer Aluminiumfolie kaschiert wurden. Außerdem sind die Folien bzw. Papiere mit Silikon beschichtet, um ihnen die wiederablösbaren Eigenschaften zu verleihen. Diese Materialien werden in einer Dicke von 0,02 - 0,30 mm verwendet. Mit Ausnahme der Polyesterfolien können sie auch als ablösbare Zwischenschichten verwendet werden. Dies ist dann erforderlich, wenn die Elastizitätseigenschaften von der wiederablösbaren Schutzschicht und der Rückschicht zu gering sind, so daß beim Aufwickeln des Laminates aus Rückschicht, wirkstoffhaltiger, wasserunlöslicher Klebmasse und wiederablösbarer Schutzschicht nach Beschichtung und Trocknung Falten im Wirkstoffpflaster entstehen würden.

Geeignete Wirkstoffe gemäß der Erfindung sind 17 β-Estradiol und 17 α-Estradiol bzw. ihre Derivate.

Unter den pharmazeutisch unbedenklichen Derivaten gemäß der Erfindung werden u.a. Ester, Ether, Ethynylverbindungen des Estradiols verstanden, wie z.B.
Estradiol (17β)-17-Butyrylacetat,
Estradiol 17 β-cipionat,
Estradiol 3,17 β-dienantat,
Estradiol 3,17 β-dipropionat,
Estradiolenantat,
Estradiol 3-hydrogensulfat (Natriumsalz),
Estradiol 17 β-(3-phenylpropionat),
Estradiolundexylat,
Estradiolvalerat,
Estradiol 17 α-(3-oxohexonat),
Epimestrol,
Quinestrol
Quinestradol,
Ethynylestradiol,
Fosferol, sowie
Estratriol.

Außerdem kommt als Oestrogen auch Chlorotrianisen in Frage.

Zu den geeigneten Gestagenen gemäß der Erfindung zählen beispielsweise
Lynestrenol,
Norethisteron,
Hydroxyprogesteron,
Medrogeston,
Progesteron,
Medroxyprogesteron,
Gestonoron,
Dydrogesteron,
Chlormadinon,
Allylestrenol,
Megestrol.

Das Verfahren zur Herstellung des Wirkstoffpflasters wird in der Weise durchgeführt, daß alle Bestandteile der wirkstoffhaltigen Klebemasse unter Rühren bzw. Kneten homogenisiert werden, ggf. unter Zusatz von organischen Lösungsmitteln zum Zwecke des Auflösens des Wirkstoffes. Die so erhaltene wirkstoffhaltige Kleberlösung bzw. -suspension wird auf die wiederablösbare Schutzschicht, die Rückschicht oder die ablösbare Zwischenschicht beschichtet, und das Lösemittel bei erhöhter Temperatur und/oder vermindertem Druck herausgetrocknet.

Auf den resultierenden wirkstoffhaltigen Klebefilm wird die Rückschicht oder die ablösbare Schutzschicht oder die wiederablösbare Zwischenschicht aufkaschiert.

Die nach dem Beschichten und Trocknen erhaltenen Breitrollen des vollständig aufgebauten Wirkstoffpflastermaterials werden in Schmalrollen aufgeschnitten und dann daraus die einzelnen Wirkstoffpflaster gestanzt. Die Herstellung der einzelnen Wirkstoffpflaster kann aber auch durch Formatstanzen aus den Breitrollen erfolgen.

Die Form der Wirkstoffpflaster kann beliebig sein, wie z.B. rund, oval, elliptisch, quadratisch oder rechteckig mit abgerundeten Ecken. Die Größe der Wirkstoffpflaster hängt von den therapeutischen Erfordernissen ab; sie kann variieren von 1 - 50 cm².

Die Erfindung wird anhand folgender Beispiele erläutert:

### Beispiel 1:

182,342 g vernetzbarer Haftkleber auf Basis von Homo- und/oder Copolymeren mit mindestens einem Derivat der Acryl- oder Methacrylsäure mit Vernetzer (z.B. Durotak 280-2516),
1,64 g Galaktomannan (z.B. Meyprogat 90),
1,60 g Propandiol-1,2 und
2,00 g Estradiol werden unter Zugabe von
8,773 g Ethanol und
8,773 g Essigsäureethylester in einem Becherglas durch Rühren homogenisiert.

Diese Masse wird mit einer Streichrakel auf eine einseitig mit Aluminium bedampfte und beidseitig mit Silikon beschichtete 100 µm dicke Polyesterfolie ausgestrichen und 10 Minuten bei 50°C im Umlufttrockenschrank getrocknet, so dass ein wirkstoffhaltiger Klebefilm mit einem Flächengewicht von 80 g/m² resultiert. Dieser wird anschließend mit einer 15µm dicken Polyesterfolie abgedeckt. Danach werden Einzelpflaster mit einer Fläche von 16 cm² ausgestanzt.

### Wirkstofffreisetzung

Zur Messung der Wirkstofffreisetzung werden 5 cm² große Pflasterabschnitte verwendet.

Auf der Seite der Rückschicht wird das Wirkstoffpflaster mit einer 100 µm dicken Polyesterfolie verklebt und nach Abziehen der wiederablösbaren Schutzschicht in 80 ml demineralisiertes Wasser von 34°C gegeben. Nach 2, 4, 6 und 24 Stunden wird das demineralisierte Wasser gewechselt und der Estradiolgehalt in den Probelösungen flüssigchromatographisch bestimmt.

Die Ergebnisse sind in Tabelle 1 wiedergegeben.

Die weiteren Beispiele unterscheiden sich in dem eingesetzten Haftkleber. Die Ergebnisse der Wirkstofffreisetzung sind in Tabelle 1 zusammengefasst.

### Beispiel 2

148,00 g nichtvernetzbarer Haftkleber auf Basis von Homo- und Copolymeren mit mindestens einem Derivat der Acryl- oder Methacrylsäure (z.B. Durotak 280-2287),
2,40 g Galaktomannan (z.B. Meyprogat 90),
1,60 g Propandiol-1,2 und
2,00 g Estradiol werden unter Zugabe von
30,667 g Ethanol und
15,337 g Essigsäureethylester in einem Becherglas durch Rühren homogenisiert.

Die Weiterverarbeitung erfolgt wie in Beispiel 1 beschrieben.

### Beispiel 3

140,26 g vernetzbarer Haftkleber auf der Basis von Homo- und Copolymeren mit mindestens einem Derivat der Acryl- oder Methacrylsäure mit Vernetzer (z.B. Durotak 126-1050)
1,64 g Galaktomannan (z.B. Meyprogat 90),
1,60 g Propandiol-1,2 und
2,00 g Estradiol werden unter Zugabe von
20 ml Ethanol und
20 ml Essigsäureethylester in einem Becherglas durch Rühren homogenisiert.

Die Weiterverarbeitung erfolgt wie in Beispiel 1 beschrieben.

### Beispiel 4

155,43 g vernetzbarer Haftkleber auf der Basis von Homo- und Copolymeren mit mindestens einem Derivat der Acryl- oder Methacrylsäure mit Vernetzer (z.B. Durotak 380-1054),
1,64 g Galaktomannan (z.B. Meyprogat 90),
1,60 g Propandiol-1,2 und
2,00 g Estradiol werden in einem Becherglas unter Rühren homogenisiert.

Die Weiterverarbeitung erfolgt wie in Beispiel 1 beschrieben.

### Beispiel 5

153,20 g vernetzbarer Haftkleber auf der Basis von Homo- und Copolymeren mit mindestens einem Derivat der Acryl- oder Methacrylsäure mit Vernetzer (z.B. Durotak 180-1197B),
1,64 g Galaktomannan (z.B. Meyprogat 90),
1,60 g Propandiol-1,2 und
2,00 g Estradiol werden unter Zugabe von
20,00 ml Ethanol und
20,00 ml Essigsäureethylester in einem Becherglas unter Rühren homogenisiert.

Die Weiterverarbeitung erfolgt wie in Beispiel 1 beschrieben.

### Beispiel 6

146,88 g vernetzbarer Haftkleber auf dr Basis von Homo- und Copolymeren mit mindestens einem Derivat der
Acryl- oder Methacrylsäure mit Vernetzer (z.B. Aroset 1880-Z-46),
1,64 g Galaktomannan (z.B. Meyprogat 90),
1,60 g Propandiol-1,2 und
2,00 g Estradiol werden unter Zugabe von
20,00 ml Ethanol und
20,00 ml Essigsäureethylester in einem Becherglas unter Rühren homogenisiert.

Die Weiterverarbeitung erfolgt wie in Beisspiel 1 beschrieben.

### Beispiel 7

139,22 g vernetzbarer Haftkleber auf der Basis von Homo- und Copolymeren mit mindestens einem Derivat der Acryl- oder Methacrylsäure mit Vernetzer (z.B. Aroset 1930-TH-46),
1,64 g Galaktomannan (z.B. Meyprogat 90),
1,60 g Propandiol-1,2 und
2,00 g Estradiol werden unter Zugabe von
30,00 ml Ethanol und
30,00 ml Essigsäureethylester in einem Becherglas unter Rühren homogenisiert.

Die Weiterverarbeitung erfolgt wie in Beispiel 1 beschreiben.

**Tabelle 1**

| Stand der Technik nach EP 0186019 - Beispiel 3 C Wirkstofffreisetzung: 0,63 mg/16 cm² x 24 Std. | | | | | |
|---|---|---|---|---|---|
| Beispiel | Wirkstofffreisetzung mg/16 cm² nach | | | | |
| | 2 h | 4 h | 6 h | 8 h | 24 h |
| 1 | 0,67 | 1,10 | 1,63 | -- | 2,38 |
| 2 | 0,77 | 1,24 | 1,85 | -- | 2,67 |
| 3 | 0,88 | 1,37 | -- | 1,63 | 2,87 |
| 4 | 0,74 | 1,19 | -- | 1,77 | 2,43 |
| 5 | 0,62 | 1,01 | -- | 1,49 | 2,28 |
| 6 | 0,66 | 1,00 | -- | 1,51 | 2,36 |
| 7 | 0,81 | 1,28 | -- | 1,91 | 2,71 |

Wie die Ergebnisse in Tabelle 1 zeigen, ist die Wirkstofffreisetzung der erfindungsgemäßen Pflaster schon nach 2 Stunden so hoch wie sie nach dem Stand der Technik erst nach 24 Stunden ist.

## Patentansprüche (Patentansprüche für folgende(n) Vertragsstaat(en): AT, BE, CH, DE, DK, FR, GB, IT, LU, NL, SE)

1. Wirkstoffpflaster für die kontrollierte Abgabe von Wirkstoffen an die Haut, welches aus einer Rückschicht und einem damit verbundenen, wasserunlöslichen Klebefilm aus einem Haftkleber besteht, der in Wasser quellbare Polymere enthält und in dem der Wirkstoff zumindest teilweise löslich ist, und aus einer den Klebefilm abdeckenden, wieder ablösbaren Schutzschicht, dadurch gekennzeichnet, daß der Haftkleber auf Homo- und/oder Copolymeren mit mindestens einem Derivat der Acryl- oder Methacrylsäure basiert und als im Kleber teilweise oder vollständig gelöste Wirkstoffe in einer Konzentration von 0,5 bis 10,0 Gew.-% Oestrogene und ihre pharmazeutisch unbedenklichen Derivate alleine oder in Kombination mit Gestagenen enthält, wobei die in Wasser quellbaren Polymeren eine Wasserlöslichkeit ≤ 0,1 Gew.-% besitzen und / oder Galaktomannane, mikrokristalline Cellulose, Polyglycoside, feinpulverisierte Polyamide, wasserlösliches Polyacrylamid, Carboxyvinylpolymerisate, agar-ähnliche Algenprodukte, Mischpolymerisate aus Methylvinylether und Maleinsäureanhydrid, Dextrin, mikrobiologisch gewonnenes Polysaccharid-Gummi, Ficoll, Methylglucosederivate, Hydroxypropylguar-Gummi, Hydroxymethylpropylcellulose, Polygalacturonsäurederivate wie Pectin und Pectinamid, sind.

2. Wirkstoffpflaster nach Anspruch 1, dadurch gekennzeichnet, daß der Haftkleber vernetzbar ist.

3. Wirkstoffpflaster nach Anspruch 1 oder 2, dadurch gekennzeichnet, daß der Haftkleber ein Lösemittelhaftkleber ist.

4. Wirkstoffpflaster nach einem der Ansprüche 1 bis 3, dadurch gekennzeichnet, daß der Haftkleber ein Dispersionshaftkleber ist.

5. Wirkstoffpflaster nach einem der Ansprüche 1 bis 3, dadurch gekennzeichnet, daß der Haftkleber ein Schmelzhaftkleber ist.

6. Wirkstoffpflaster nach einem der Ansprüche 1 bis 5, dadurch gekennzeichnet, daß der Haftkleber mindestens ein klebrigmachendes Harz in einer Menge von 0,5 bis 50 Gew.-%, vorzugsweise 1 bis 20 Gew.-%, enthält.

7. Wirkstoffpflaster nach einem der Ansprüche 1 bis 6, dadurch gekennzeichnet, daß der Klebefilm die Kristallisation des Wirkstoffes verzögernde oder verhindernde Stoffe in einem Anteil von 0,1 bis 20 Gew.-%, vorzugsweise 0,5 bis 10 Gew.-% enthält.

8. Wirkstoffpflaster nach einem der Ansprüche 1 bis 7, dadurch gekennzeichnet, daß der Klebefilm in Wasser quellbare Polymere in einem Anteil von 0,01 bis 10 Gew.-%, vorzugsweise 0,1 bis 5 Gew.-% enthält.

9. Wirkstoffpflaster nach einem der Ansprüche 1 bis 8, dadurch gekennzeichnet, daß der wirkstoffhaltige Klebefilm eine Dicke von 0,01 bis 0,3 mm, vorzugsweise 0,04 bis 0,20 mm aufweist.

10. Verfahren zur Herstellung von Wirkstoffpflastern nach einem der Ansprüche 1 bis 9, dadurch gekennzeichnet, daß die homogenisierte, wirkstoffhaltige Klebmasse auf die wieder ablösbare Schutzschicht aufgetragen und nach Abdampfen des Lösungsmittels mit der für den Wirkstoff undurchlässigen Rückschicht abgedeckt wird.

11. Verfahren zur Herstellung von Wirkstoffpflastern nach einem der Ansprüche 1 bis 10, dadurch gekennzeichnet, daß die wirkstoffhaltige Klebmasse auf eine ablösbare Zwischenschicht aufgetragen und nach Abdampfen des Lösungsmittels mit der für den Wirkstoff undurchlässigen Rückschicht abgedeckt wird.

12. Verfahren zur Herstellung von Wirkstoffpflastern nach Anspruch 11, dadurch gekennzeichnet, daß die ablösbare Zwischenschicht in einem späteren Arbeitsgang entfernt und durch die wieder ablösbare Schutzschicht ersetzt wird.

13. Verfahren zur Herstellung von Wirkstoffpflastern nach einem der Ansprüche 1 bis 9, dadurch gekennzeichnet, daß die wirkstoffhaltige Klebmasse auf die für den Wirkstoff undurchlässige Rückschicht aufgetragen und nach Abdampfen des Lösungsmittels mit der wieder ablösbaren Schutzschicht oder Zwischenschicht abgedeckt wird.

14. Verfahren zur Herstellung von Wirkstoffpflastern nach einem der Ansprüche 1 bis 13, dadurch gekennzeichnet, daß die Vereinzelung der Wirkstoffpflaster durch Schneiden und/oder Formatstanzen erfolgt.

## Patentansprüche (Patentansprüche für folgende(n) Vertragsstaat(en): ES)

1. Verfahren zur Herstellung eines Wirkstoffpflasters für die kontrollierte Abgabe von Wirkstoffen an die Haut, welches aus einer Rückschicht und einem damit verbundenen wasserunlöslichen Klebefilm aus einem Haftkleber besteht, der in Wasser quellbare Polymere enthält und in dem der Wirkstoff zumindest teilweise löslich ist, und aus einer der Klebefilm abdeckenden, wieder ablösbaren Schutzschicht, dadurch gekennzeichnet, dass eine Klebemasse auf Basis eines Homo- und/oder Copolymeren mit mindestens einem Derivat der Acryl- oder Methacrylsäure homogenisiert wird, wobei als vorher oder gleichzeitig zugesetzter Wirkstoff Oestrogene und ihre pharmazeutisch unbedenklichen Derivate alleine oder in Kombination mit Gestagenen eingesetzt werden, dass die Masse auf die wiederablösbare Schutzschicht oder eine ablösbare Zwischenschicht aufgetragen und nach Abdampfen des Lösungsmittels mit der für den Wirkstoff undurchlässigen Rückschicht abgedeckt wird, wobei als in Wasser quellbare Polymere solche mit einer Wasserlöslichkeit ≤ 0,1 Gew.-% und / oder Galaktomannane, mikrokristalline Cellulose, Polyglycoside, feinpulverisierte Polyamide, wasserlösliches Polyacrylamid, Carboxyvinylpolymerisate, agar-ähnliche Algenprodukte, Mischpolymerisate aus Methylvinylether und Maleinsäureanhydrid, Dextrin, mikrobiologisch gewonnenes Polysaccharid-Gummi, Ficoll, Methylglucosederivate, Hydroxypropylguar-Gummi, Hydroxymethylpropylcellulose, Polygalacturonsäurederivate wie Pectin und Pectinamid, eingesetzt werden.

2. Verfahren nach Anspruch 1, dadurch gekennzeichnet, dass die wirkstoffhaltige Klebemasse auf die für den Wirkstoff undurchlässige Rückschicht aufgetragen und nach Abdampfen des Lösungsmittels mit der wieder ablösbaren Schutzschicht oder Zwischenschicht abgedeckt wird.

3. Verfahren nach Anspruch 1 oder 2, dadurch gekennzeichnet, dass die im Kleber teilweise oder vollständig gelösten Wirkstoffe in einer Konzentration von 0,5 bis 10,0 Gew.-% eingesetzt werden.

4. Verfahren nach einem der Ansprüche 1 bis 3, dadurch gekennzeichnet, daß ein vernetzbarer Haftkleber eingesetzt wird.

5. Verfahren nach einem der Ansprüche 1 bis 4, dadurch gekennzeichnet, daß als Haftkleber ein Lösemittel-, Dispersions- oder Schmelzhaftkleber eingesetzt wird.

6. Verfahren nach einem der Ansprüche 1 bis 5, dadurch gekennzeichnet, daß als Haftkleber ein Kleber mit mindestens einem klebrigmachenden Harz in einer Menge von 0,5 bis 50 Gew.-%, vorzugsweise 1 bis 20 Gew.-%, eingesetzt wird.

7. Verfahren nach einem der Ansprüche 1 bis 6, dadurch gekennzeichnet, daß in den Klebefilm die Kristallisation des Wirkstoffes verzögernde oder verhindernde Stoffe in einem Anteil von 0,1 bis 20 Gew.-%, vorzugsweise 0,5 bis 10 Gew.-%, eingebracht werden.

8. Verfahren nach einem der Ansprüche 1 bis 7, dadurch gekennzeichnet, daß ein Klebefilm mit einem Anteil von 0,01 bis 10 Gew.-%, vorzugsweise 0,1 bis 5 Gew.-%, von in Wasser quellbaren Polymeren eingesetzt wird.

9. Verfahren nach einem der Ansprüche 1 bis 8, dadurch gekennzeichnet, daß der wirkstoffhaltige Klebefilm in einer Dicke von 0,01 bis 0,3 mm, vorzugsweise 0,04 bis 0,20 mm, eingesetzt wird.

10. Verfahren nach einem der Ansprüche 1 bis 9, dadurch gekennzeichnet, daß die Vereinzelung der Wirkstoffpflaster durch Schneiden und/oder Formatstanzen erfolgt.

11. Verfahren nach einem der Ansprüche 1 bis 10, dadurch gekennzeichnet, daß die ablösbare Zwischenschicht in einem späteren Arbeitsgang entfernt und durch die wieder ablösbare Schutzschicht ersetzt wird.

## Patentansprüche (Patentansprüche für folgende(n) Vertragsstaat(en): GR)

1. Verfahren zur Herstellung eines Wirkstoffpflasters für die kontrollierte Abgabe von Wirkstoffen an die Haut, welches aus einer Rückschicht und einem damit verbundenen, wasserunlöslichen Klebefilm aus einem Haftkleber besteht, der in Wasser quellbare Polymere enthält und in dem der Wirkstoff zumindest teilweise löslich ist, und aus einer der Klebefilm abdeckenden, wieder ablösbaren Schutzschicht, dadurch gekennzeichnet, dass eine Klebemasse auf Basis eines Homo- und/oder Copolymeren mit mindestens einem Derivat der Acryl- oder Methacrylsäure homogenisiert wird, wobei als vorher oder gleichzeitig zugesetzter Wirkstoff Oestrogene und ihre pharmazeutisch unbedenklichen Derivate alleine oder in Kombination mit Gestagenen eingesetzt werden, dass die Masse auf die wiederablösbare Schutzschicht oder eine ablösbare Zwischenschicht aufgetragen und nach Abdampfen des Lösungsmittels mit der für den Wirkstoff undurchlässigen Rückschicht abgedeckt wird, wobei als in Wasser quellbare Polymere solche mit einer Wasserlöslichkeit ≤ 0,1 Gew.-% und / oder Galaktomannane, mikrokristalline Cellulose, Polyglycoside, feinpulverisierte Polyamide, wasserlösliches Polyacrylamid, Carboxyvinylpolymerisate, agar-ähnliche Algenprodukte, Mischpolymerisate aus Methylvinylether und Maleinsäureanhydrid, Dextrin, mikrobiologisch gewonnenes Polysaccharid-Gummi, Ficoll, Methylglucosederivate, Hydroxypropylguar-Gummi, Hydroxymethylpropylcellulose, Polygalacturonsäurederivate wie Pectin und Pectinamid, eingesetzt werden.

2. Verfahren nach Anspruch 1, dadurch gekennzeichnet, dass die wirkstoffhaltige Klebemasse auf die für den Wirkstoff undurchlässige Rückschicht aufgetragen und nach Abdampfen des Lösungsmittels mit der wieder ablösbaren Schutzschicht oder Zwischenschicht abgedeckt wird.

3. Verfahren nach Anspruch 1 oder 2, dadurch gekennzeichnet, dass die im Kleber teilweise oder vollständig gelösten Wirkstoffe in einer Konzentration von 0,5 bis 10,0 Gew.-% eingesetzt werden.

4. Verfahren nach einem der Ansprüche 1 bis 3, dadurch gekennzeichnet, daß ein vernetzbarer Haftkleber eingesetzt wird.

5. Verfahren nach einem der Ansprüche 1 bis 4, dadurch gekennzeichnet, daß als Haftkleber ein Lösemittel-, Dispersions- oder Schmelzhaftkleber eingesetzt wird.

6. Verfahren nach einem der Ansprüche 1 bis 5, dadurch gekennzeichnet, daß als Haftkleber ein Kleber mit mindestens einem klebrigmachenden Harz in einer Menge von 0,5 bis 50 Gew.-%, vorzugsweise 1 bis 20 Gew.-%, eingesetzt wird.

7. Verfahren nach einem der Ansprüche 1 bis 6, dadurch gekennzeichnet, daß in den Klebefilm die Kristallisation des Wirkstoffes verzögernde oder verhindernde Stoffe in einem Anteil von 0,1 bis 20 Gew.-%, vorzugsweise 0,5 bis 10 Gew.-%, eingebracht werden.

8. Verfahren nach einem der Ansprüche 1 bis 7, dadurch gekennzeichnet, daß ein Klebefilm mit einem Anteil von 0,01 bis 10 Gew.-%, vorzugsweise 0,1 bis 5 Gew.-%, von in Wasser quellbaren Polymeren eingesetzt wird.

9. Verfahren nach einem der Ansprüche 1 bis 8, dadurch gekennzeichnet, daß der wirkstoffhaltige Klebefilm in einer Dicke von 0,01 bis 0,3 mm, vorzugsweise 0,04 bis 0,20 mm, eingesetzt wird.

10. Verfahren nach einem der Ansprüche 1 bis 9, dadurch gekennzeichnet, daß die Vereinzelung der Wirkstoffpflaster durch Schneiden und/oder Formatstanzen erfolgt.

11. Verfahren nach einem der Ansprüche 1 bis 10, dadurch gekennzeichnet, daß die ablösbare Zwischenschicht in einem späteren Arbeitsgang entfernt und durch die wieder ablösbare Schutzschicht ersetzt wird.

## Claims (Claims for the following Contracting State(s): AT, BE, CH, DE, DK, FR, GB, IT, LU, NL, SE)

1. Active substance plaster for the controlled release of active substances to the skin consisting of a backing layer, an adhesive film connected therewith, which film is water-insoluble and consists of a pressure-sensitive adhesive which comprises water-swellable polymers and in which the active substance is at least partially soluble, and of a removable protective layer covering the adhesive film, characterized in that said pressure-sensitive adhesive is based on homopolymers and/or copolymers with at least one derivative of the acrylic or methacrylic acid, and that said pressure-sensitive adhesive comprises as active substances, at a concentration of 0.5 to 10.0%-wt, estrogens and their pharmaceutically acceptable derivatives alone or in combination with gestagens, said active substances being partially or completely dissolved in said adhesive, and wherein the water-swellable polymers have a solubility in water ≤ 0.1%-wt and/or wherein the water-swellable polymers are galactomannans, microcrystalline cellulose, polyglycosides, finely pulverized polyamides, water-soluble polyacrylamide, carboxyinyl polymers, seaweed products similar to agar, copolymers of methylvinyl ether and maleic acid anhydride, dextrin, microbiologically recovered polysaccharide gum, Ficoll, methylglucose derivatives, hydroxypropyl guar gum, hydroxymethylpropyl cellulose, polygalacturonic acid derivatives such as pectin and pectinamide.

2. Active substance plaster according to claim 1, characterized in that the pressure-sensitive adhesive is cross-linkable.

3. Active substance plaster according to claims 1 or 2, characterized in that the pressure-sensitive adhesive is a pressure-sensitive adhesive solution.

4. Active substance plaster according to any one of claims 1 to 3, characterized in that the pressure-sensitive adhesive is a pressure-sensitive adhesive dispersion.

5. Active substance plaster according to any one of claims 1 to 3, characterized in that the pressure-sensitive adhesive is a hot-melt pressure-sensitive adhesive.

6. Active substance plaster according to any one of claims 1 to 5, characterized in that the pressure-sensitive adhesive comprises at least one tackifying resin in an amount of 0.5 to 50%-wt, preferably 1 to 20%-wt.

7. Active substance plaster according to any one of claims 1 to 6, characterized in that the adhesive film comprises substances delaying or preventing the crystallization of the active substance at a proportion of 0.1 to 20%-wt, preferably 0.5 to 10%-wt.

8. Active substance plaster according to any one of claims 1 to 7, characterized in that the adhesive film comprises water-swellable polymers at a proportion of 0.01 to 10%-wt, preferably 0.1 to 5%-wt.

9. Active substance plaster according to any one of claims 1 to 8, characterized in that the active substance-containing adhesive film has a thickness of 0.01 to 0.3 mm, preferably 0.04 to 0.20 mm.

10. A process for the production of active substance plasters as defined in any one of claims 1 to 9, characterized in that the homogenized, active substance-containing adhesive mass is applied onto the removable protective layer and, after evaporation of the solvent, is covered with the backing layer, which is impermeable to the active substance.

11. A process for the production of active substance plasters as defined in any one of claims 1 to 10, characterized in that the active substance-containing adhesive mass is applied to a removable intermediate layer and, after evaporation of the solvent, covered with the backing layer, which is impermeable to the active substance.

12. A process for the production of active substance plasters according to claim 11, characterized in that the removable intermediate layer is removed in a later process step and replaced by the removable protective layer.

13. A process for the production of active substance plasters as defined in any one of claims 1 to 9, characterized in that the active substance-containing adhesive mass is applied to the backing layer, which is impermeable to the active substance, and, after evaporation of the solvent, is covered with the removable protective layer or with the intermediate layer.

14. A process for the production of active substance plasters as defined in any one of claims 1 to 13, characterized in that separation of the active substance plasters is carried out by cutting and/or format punching.

## Claims (Claims for the following Contracting State(s): ES)

1. Process for the production of an active substance plaster for the controlled release of active substances to the skin consisting of a backing layer, an adhesive film connected therewith, which film is water-insoluble and consists of a pressure-sensitive adhesive which comprises water-swellable polymers and in which the active substance is at least partially soluble, and of a removable protective layer covering the adhesive film, characterized in that an adhesive mass based on a homopolymer and/or copolymer is homogenized with at least one derivative of acrylic or methacrylic acid, whereby as active substance, which is added prior thereto or simultaneously, there are used estrogens and their pharmaceutically acceptable derivatives, alone or in combination with gestagens, that the mass is applied to the removable protective layer or to a removable intermediate layer and, after evaporation of the solvent, is covered with the backing layer, which is impermeable to the active substance, and wherein the water-swellable polymers used in the process are water-swellable polymers having a solubility in water ≤ 0.1%-wt, and/or wherein galacto-mannans, microcrystalline cellulose, polyglycosides, finely pulverized polyamides, water-soluble polyacrylamide, carboxyinyl polymers, seaweed products similar to agar, copolymers of methylvinyl ether and maleic acid anhydride, dextrin, microbiologically recovered polysaccharide gum, Ficoll, methylglucose derivatives, hydroxypropyl guar gum, hydroxymethylpropyl cellulose, polygalacturonic acid derivatives, such as pectin and pectinamide, are used as water-swellable polymers.

2. The process according to claim 1, characterized in that the active substance-containing adhesive mass is applied to the backing layer, which is impermeable to the active substance, and, after evaporation of the solvent, is covered with the the removable protective layer or intermediate layer.

3. The process according to claim 1 or 2, characterized in that the active substances, which are partially or completely dissolved in the adhesive, are used in a concentration of 0.5 to 10.0%-wt.

4. The process according to any one of claims 1 to 3, characterized in that a cross-linkable pressure-sensitive adhesive is used.

5. The process according to any one of claims 1 to 4, characterized in that as pressure-sensitive adhesive a pressure-sensitive adhesive solution, a pressure-sensitive adhesive dispersion or a pressure-sensitive hot-melt adhesive is used.

6. The process according to any one of the claims 1 to 5, characterized in that as pressure-sensitive adhesive an adhesive containing at least one tackifying resin in an amount of 0.5 to 50%-wt., preferably 1 to 20%-wt., is used.

7. The process according to any one of claims 1 to 6, characterized in that in the adhesive film there are incorporated substances delaying or preventing the crystallization of the active substance at a proportion of 0.1 to 20%-wt, preferably 0.5 to 10%-wt.

8. The process according to any one of claims 1 to 7, characterized in that an adhesive film is employed which comprises water-swellable polymers at a proportion of 0.01 to 10%-wt, preferably 0.1 to 5%-wt.

9. The process according to any one of claims 1 to 8, characterized in that the active substance-containing adhesive film which is used has a thickness of 0.01 to 0.3 mm, preferably 0.04 to 0.20 mm.

10. The process according to any one of claims 1 to 9, characterized in that separation of the active substance plasters is carried out by cutting and/or format punching.

11. The process according to any one of claims 1 to 10, characterized in that the removable backing layer is removed in a later process step and replaced by the removable protective layer.

## Claims (Claims for the following Contracting State(s): GR)

1. Process for the production of an active substance plaster for the controlled release of active substances to the skin consisting of a backing layer, an adhesive film connected therewith, which film is water-insoluble and consists of a pressure-sensitive adhesive which comprises water-swellable polymers and in which the active substance is at least partially soluble, and of a removable protective layer covering the adhesive film, characterized in that an adhesive mass based on a homopolymer and/or copolymer is homogenized with at least one derivative of acrylic or methacrylic acid, whereby as active substance, which is added prior thereto or simultaneously, there are used estrogens and their pharmaceutically acceptable derivatives, alone or in combination with gestagens, that the mass is applied to the removable protective layer or to a removable intermediate layer and, after evaporation of the solvent, is covered with the backing layer, which is impermeable to the active substance, and wherein the water-swellable polymers used in the process are water-swellable polymers having a solubility in water ≤ 0.1%-wt, and/or wherein galacto-mannans, microcrystalline cellulose, polyglycosides, finely pulverized polyamides, water-soluble polyacrylamide, carboxyvinyl polymers, seaweed products similar to agar, copolymers of methylvinyl ether and maleic acid anhydride, dextrin, microbiologically recovered polysaccharide gum, Ficoll, methylglucose derivatives, hydroxypropyl guar gum, hydroxymethylpropyl cellulose, polygalacturonic acid derivatives, such as pectin and pectinamide, are used as water-swellable polymers.

2. The process according to claim 1, characterized in that the active substance-containing adhesive mass is applied to the backing layer, which is impermeable to the active substance, and, after evaporation of the solvent, is covered with the the removable protective layer or intermediate layer.

3. The process according to claim 1 or 2, characterized in that the active substances, which are partially or completely dissolved in the adhesive, are used in a concentration of 0.5 to 10.0%-wt.

4. The process according to any one of claims 1 to 3, characterized in that a cross-linkable pressure-sensitive adhesive is used.

5. The process according to any one of claims 1 to 4, characterized in that as pressure-sensitive adhesive a pressure-sensitive adhesive solution, a pressure-sensitive adhesive dispersion or a pressure-sensitive hot-melt adhesive is used.

6. The process according to any one of, the claims 1 to 5, characterized in that as pressure-sensitive adhesive an adhesive containing at least one tackifying resin in an amount of 0.5 to 50%-wt., preferably 1 to 20%-wt., is used.

7. The process according to any one of claims 1 to 6, characterized in that in the adhesive film there are incorporated substances delaying or preventing the crystallization of the active substance at a proportion of 0.1 to 20%-wt, preferably 0.5 to 10%-wt.

8. The process according to any one of claims 1 to 7, characterized in that an adhesive film is employed which comprises water-swellable polymers at a proportion of 0.01 to 10%-wt, preferably 0.1 to 5%-wt.

9. The process according to any one of claims 1 to 8, characterized in that the active substance-containing adhesive film which is used has a thickness of 0.01 to 0.3 mm, preferably 0.04 to 0.20 mm.

10. The process according to any one of claims 1 to 9, characterized in that separation of the active substance plasters is carried out by cutting and/or format punching.

11. The process according to any one of claims 1 to 10, characterized in that the removable backing layer is removed in a later process step and replaced by the removable protective layer.

## Revendications (Revendications pour l'(les) Etat(s) contractant(s) suivant(s): AT, BE, CH, DE, DK, FR, GB, IT, LU, NL, SE)

1. Timbre de substance active pour le transfert réglé de principes actifs à la peau, qui se compose d'une couche dorsale et d'un film collant insoluble dans l'eau, qui y est lié, formé d'une colle de contact ou autoadhésive, qui contient des polymères gonflables à l'eau et dans lequel le principe actif est au moins partiellement soluble et d'une couche protectrice, amovible, recouvrant le film de colle, caractérisé en ce que la colle autoadhésive ou de contact se base sur un homopolymère et/ou un copolymère avec au moins un dérivé de l'acide acrylique ou de l'acide méthacrylique et contient des oestrogènes et leurs dérivés pharmaceutiquement acceptables, seul ou en combinaison avec des gestagènes, sous forme de principes actifs partiellement ou totalement dissous dans la colle, en une concentration de 0,5 à 10,0% en poids,
les polymères gonflables à l'eau ayant une solubilité dans l'eau de ≤ 0,1% en poids, et/ou étant du galactomannane, de la cellulose microcristallisée, des polyglycosides, des polyamides finement pulvérisés, du polyacrylamide soluble dans l'eau, des polymères carboxyvinyliques, des produits d'algues analogues à la gélose, des copolymères d'éther méthylvinylique et d'anhydride maléique, de la dextrine, des gommes de polysaccharides que l'on obtient par voie microbiologique, du Ficoll, des dérivés du méthylglucose, de la gomme de l'hydroxypropylguar, l'hydroxyméthylpropylcellulose, des dérivés de polyacidegalacturonique, comme la pectine ou le pectinamide.

2. Timbre de substance active suivant la revendication 1, caractérisé en ce que la colle de contact est réticulable.

3. Timbre de substance active suivant la revendication 1 ou 2, caractérisé en ce que la colle de contact est une colle de contact à solvant.

4. Timbre de substance active suivant l'une quelconque des revendications 1 à 3, caractérisé en ce que la colle de contact est une colle de contact à dispersion.

5. Timbre de substance active suivant l'une quelconque des revendications 1 à 3, caractérisé en ce que la colle de contact est une colle de contact fusible.

6. Timbre de substance active suivant l'une quelconque des revendications 1 à 5, caractérisé en ce que la colle de contact contient au moins une résine conférant de l'adhésivité en une proportion de 0,5 à 50% en poids, de préférence, 1 à 20% en poids.

7. Timbre de substance active suivant l'une quelconque des revendications 1 à 6, caractérisé en ce que le film de colle contient des substances empêchant ou retardant la cristallisation du principe actif en une proportion de 0,1 à 20% en poids, de préférence, 0,5 à 10% en poids.

8. Timbre de substance active suivant l'une quelconque des revendications 1 à 7, caractérisé en ce que le film de colle contient des polymères gonflables dans l'eau en une proportion de 0,01 à 10% en poids, de préférence, 0,1 à 5% en poids.

9. Timbre de substance active suivant l'une quelconque des revendications 1 à 8, caractérisé en ce que le film de colle contenant le principe actif présente une épaisseur de 0,01 à 0,3 mm, de préférence, 0,04 à 0,20 mm.

10. Procédé de fabrication de timbres de substance active suivant l'une quelconque des revendications 1 à 9, caractérisé en ce que l'on applique la masse de colle contenant le principe actif, homogénéisée sur la couche protectrice amovible et, après évaporation du solvant, on la recouvre de la couche dorsale imperméable au principe actif.

11. Procédé de fabrication de timbres de substance active suivant l'une quelconque des revendications 1 à 10, caractérisé en ce que l'on applique la masse de colle contenant le principe actif sur une couche intermédiaire amovible et, après évaporation du solvant, on la recouvre de la couche dorsale imperméable au principe actif.

12. Procédé de fabrication de timbres de substance active suivant la revendication 11, caractérisé en ce que l'on enlève la couche intermédiaire amovible au cours d'une opération ultérieure et on la remplace par la couche protectrice amovible.

13. Procédé de fabrication de timbres de substance active suivant l'une quelconque des revendications 1 à 9, caractérisé en ce que l'on applique la masse de colle contenant le principe actif sur la couche dorsale imperméable au principe actif et, après évaporation du solvant, on la recouvre de la couche intermédiaire ou de la couche protectrice amovible.

14. Procédé de fabrication de timbres de substance active suivant l'une quelconque des revendications 1 à 13, caractérisé en ce que l'individualisation des timbres de substance active s'effectue par découpe et/ou estampage au format.

## Revendications (Revendications pour l'(les) Etat(s) contractant(s) suivant(s): ES)

1. Procédé de fabrication d'un timbre de substance active pour le transfert réglé de principes actifs à la peau, qui se compose d'une couche dorsale et d'un film collant insoluble dans l'eau, qui y est lié, formé d'une colle de contact ou autoadhésive, qui contient des polymères gonflables à l'eau et dans lequel le principe actif est au moins partiellement soluble et d'une couche protectrice, amovible, recouvrant le film de colle, caractérisé en ce que l'on homogénéise une masse de colle à base d'un homopolymère et/ou un copolymère avec au moins un dérivé de l'acide acrylique ou de l'acide méthacrylique, où on utilise, à titre de principe actif ajouté préalablement ou simultanément, des oestrogènes et leurs dérivés pharmaceutiquement acceptables, seul ou en combinaison avec des gestagènes, on applique la masse sur la couche protectrice amovible ou une couche intermédiaire amovible et, après évaporation du solvant, on la recouvre de la couche dorsale imperméable au principe actif,
en utilisant comme polymères gonflables à l'eau ceux d'une solubilité dans l'eau de ≤ 0,1% en poids, et/ou du galactomannane, de la cellulose microcristallisée, des polyglycosides, des polyamides finement pulvérisés, du polyacrylamide soluble dans l'eau, des polymères carboxyvinyliques, des produits d'algues analogues à la gélose, des copolymères d'éther méthylvinylique et d'anhydride maléique, de la dextrine, des gommes de polysaccharides que l'on obtient par voie microbiologique, du Ficoll, des dérivés du méthylglucose, de la gomme de l'hydroxypropylguar, l'hydroxyméthylpropylcellulose, des dérivés de polyacidegalacturonique, comme la pectine ou le pectinamide.

2. Procédé suivant la revendication 1, caractérise en ce que l'on applique la masse de colle contenant le principe actif sur la couche dorsale imperméable au principe actif et, après évaporation du solvant, on la recouvre de la couche intermédiaire ou de la couche protectrice amovible.

3. Procédé suivant la revendication 1 ou 2, caractérisé en ce que l'on utilise des principes actifs partiellement ou totalement dissous dans la colle en une concentration de 0,5 à 10,0% en poids.

4. Procédé suivant l'une quelconque des revendications 1 à 3, caractérisé en ce que l'on utilise une colle de contact réticulable.

5. Procédé suivant l'une quelconque des revendications 1 à 4, caractérisé en ce que l'on utilise, à titre de colle de contact, une colle de contact à solvant, à dispersion ou fusible.

6. Procédé suivant l'une quelconque des revendications 1 à 5, caractérisé en ce que l'on utilise, à titre de colle de contact, une colle comportant au moins une résine conférant de l'adhésivité en une proportion de 0,5 à 50% en poids, de préférence 1 à 20% en poids.

7. Procédé suivant l'une quelconque des revendications 1 à 6, caractérisé en ce que l'on incorpore au film de colle, des substances empêchant ou retardant la cristallisation du principe actif en une proportion de 0,1 à 20% en poids, de préférence, 0,5 à 10% en poids.

8. Procédé suivant l'une quelconque des revendications 1 à 7, caractérisé en ce que l'on utilise un film de colle comportant une proportion de 0,01 à 10% en poids de préférence, 0,1 à 5% en poids, de polymères gonflables à l'eau.

9. Procédé suivant l'une quelconque des revendications 1 à 8, caractérisé en ce que l'on utilise le film de colle contenant le principe actif en une épaisseur de 0,01 à 0,3 mm, de préférence, 0,04 à 0,20 mm.

10. Procédé suivant l'une quelconque des revendications 1 à 9, caractérisé en ce que l'individualisation des timbres de substance active s'effectue par découpe et/ou estampage au format.

11. Procédé suivant l'une quelconque des revendications 1 à 10, caractérisé en ce que l'on enlève la couche intermédiaire amovible au cours d'une étape opératoire ultérieure et on la remplace par la couche protectrice amovible.

## Revendications (Revendications pour l'(les) Etat(s) contractant(s) suivant(s): GR)

1. Procédé de fabrication d'un timbre de substance active pour le transfert réglé de principes actifs à la peau, qui se compose d'une couche dorsale et d'un film collant insoluble dans l'eau, qui y est lié, formé d'une colle de contact ou autoadhésive, qui contient des polymères gonflables à l'eau et dans lequel le principe actif est au moins partiellement soluble et d'une couche protectrice, amovible, recouvrant le film de colle, caractérisé en ce que l'on homogénéise une masse de colle à base d'un homopolymère et/ou un copolymère avec au moins un dérivé de l'acide acrylique ou de l'acide méthacrylique, où on utilise, à titre de principe actif ajouté préalablement ou simultanément, des oestrogènes et leurs dérivés pharmaceutiquement acceptables, seul ou en combinaison avec des gestagènes, on applique la masse sur la couche protectrice amovible ou une couche intermédiaire amovible et, après évaporation du solvant, on la recouvre de la couche dorsale imperméable au principe actif,
en utilisant comme polymères gonflables à l'eau ceux d'une solubilité dans l'eau de ≤ 0,1% en poids, et/ou du galactomannane, de la cellulose microcristallisée, des polyglycosides, des polyamides finement pulvérisés, du polyacrylamide soluble dans l'eau, des polymères carboxyvinyliques, des produits d'algues analogues à la gélose, des copolymères d'éther méthylvinylique et d'anhydride maléique, de la dextrine, des gommes de polysaccharides que l'on obtient par voie microbiologique, du Ficoll, des dérivés du méthylglucose, de la gomme de l'hydroxypropylguar, l'hydroxyméthylpropylcellulose, des dérivés de polyacidegalacturonique, comme la pectine ou le pectinamide.

2. Procédé suivant la revendication 1, caractérisé en ce que l'on applique la masse de colle contenant le principe actif sur la couche dorsale imperméable au principe actif et, après évaporation du solvant, on la recouvre de la couche intermédiaire ou de la couche protectrice amovible.

3. Procédé suivant la revendication 1 ou 2, caractérisé en ce que l'on utilise des principes actifs partiellement ou totalement dissous dans la colle en une concentration de 0,5 à 10,0% en poids.

4. Procédé suivant l'une quelconque des revendications 1 à 3, caractérisé en ce que l'on utilise une colle de contact réticulable.

5. Procédé suivant l'une quelconque des revendications 1 à 4, caractérisé en ce que l'on utilise, à titre de colle de contact, une colle de contact à solvant, à dispersion ou fusible.

6. Procédé suivant l'une quelconque des revendications 1 à 5, caractérisé en ce que l'on utilise, à titre de colle de contact, une colle comportant au moins une résine conférant de l'adhésivité en une proportion de 0,5 à 50% en poids, de préférence 1 à 20% en poids.

7. Procédé suivant l'une quelconque des revendications 1 à 6, caractérisé en ce que l'on incorpore au film de colle, des substances empêchant ou retardant la cristallisation du principe actif en une proportion de 0,1 à 20% en poids, de préférence, 0,5 à 10% en poids.

8. Procédé suivant l'une quelconque des revendications 1 à 7, caractérisé en ce que l'on utilise un film de colle comportant une proportion de 0,01 à 10% en poids, de préférence, 0,1 à 5% en poids, de polymères gonflables à l'eau.

9. Procédé suivant l'une quelconque des revendications 1 à 8, caractérisé en ce que l'on utilise le film de colle contenant le principe actif en une épaisseur de 0,01 à 0,3 mm, de préférence, 0,04 à 0,20 mm.

10. Procédé suivant l'une quelconque des revendications 1 à 9, caractérisé en ce que l'individualisation des timbres de substance active s'effectue par découpe et/ou estampage au format.

11. Procédé suivant l'une quelconque des revendications 1 à 10, caractérisé en ce que l'on enlève la couche intermédiaire amovible au cours d'une étape opératoire ultérieure et on la remplace par la couche protectrice amovible.
